# EUROPEAN PATENT APPLICATION

(11) **EP 4 296 940 A1**
(43) Date of publication of application: **27.12.2023**
(21) Application number: 22180229.1
(22) Date of filing: 21.06.2022
(51) Int. Cl.: G06T 7/00, G06T 7/73, A61B 34/10, A61B 34/20, A61B 90/00

(54) **SYSTEMS AND METHODS FOR EFFORTLESS AND RELIABLE 3D NAVIGATION FOR MUSCULOSKELETAL SURGERY BASED ON SINGLE 2D X-RAY IMAGES**

(71) Applicant: metamorphosis GmbH, 33184 Altenbeken (DE)
(72) Inventor: Blau, Arno, 72336 Balingen (DE); Lamm, Artur, 33102 Paderborn (DE); Pede, Stefan, 33154 Salzkotten (DE)
(74) Representative: LKGLOBAL Lorenz & Kopf Patentanwalt Attorney at Law PartG mbB

(57) **Abstract**

A computer program product is provided including instructions which, when executed on a processing unit of a system, cause the system to receive one X-ray image, a 3D data set describing a region of interest within a patient's anatomy, and a 3D model of the surgical object. The X-ray image is a 2D projection image depicting at least part of a surgical object including an anchor point as well as the region of interest. The computer program product is configured to determine a 2D position of the anchor point in the X-ray image and an imaging direction onto the region of interest based on the X-ray image. Based on this information, a 3D position of the anchor point in a 3D coordinate system defined by the 3D data set is determined. Further, a 3D position and 3D orientation of the surgical object relative to the region of interest is determined by means of the computer program product based on the 3D model of the surgical object and the 3D position of the anchor point in the 3D coordinate system.

## Description

### FIELD OF INVENTION

The invention relates to the fields of computer- and robot-assisted surgery. Further, the invention relates to systems and methods providing information related to surgical objects and anatomy based on CT data, or another type of 3D X-ray scan, and X-ray images. In particular, the invention relates to systems and methods for automatically determining spatial position and orientation of a surgical object relative to a patient. The methods may be implemented as a computer program executable on a processing unit of the systems.

### BACKGROUND OF THE INVENTION

Spinal surgery, and in particular, spinal fusion, has become a commonly performed surgical procedure. However, surgery on the spine also poses substantial risks to the patient. An incorrect drilling performed on the spine may damage the spinal cord, which can cause serious injuries to the nerves or the covering of the spinal cord, potentially leading to chronic pain, permanent paralysis, incontinence, or sexual dysfunction. In order to mitigate these risks due to incorrect drillings, computer-assisted navigation is already used with some frequency in spinal surgery. Computer assistance concerns navigating the surgeon, ensuring that drillings are performed in the correct location, pedicle screws are properly placed, and so on. This entails determining the precise relative 3D position and 3D orientation between a surgical tool (such as a drill) and the patient (e.g., relative to a desired drilling trajectory).

Almost all existing navigation systems require additional procedural steps and apparatuses like 3D cameras, trackers, reference bodies, and so on. In navigated spinal surgery, most current systems use optical tracking, where dynamic reference frames are attached to the spine (for patient tracking) and a reference body is attached to the surgical tool. All references must then be at all times visible to a 3D camera. Such an approach has multiple disadvantages, including but not limited to:
- Plausibility and accuracy of the registration must be continuously watched. Registration may have to be repeated in case of tracker movements.
- If a tracker movement goes unnoticed, navigation instructions will be incorrect, possibly resulting in harm to the patient.
- Accuracy decreases with increasing distance from the camera.

In summary, existing navigation systems are error-prone. The long chain of errors in the navigation workflow of registration, instrument calibration and real time tracking means that navigation systems can only be as accurate as the underlying weakest part of the chain. Therefore, even when working with navigation systems, surgeons need to constantly ensure the accuracy of the navigation instructions, e.g. by tactile and landmark checking, which is an uncertain validation procedure.

Robot-assisted surgery systems are becoming more popular due to the precision they are thought to provide. However, as robots work substantially on the instructions provided by navigation systems, robots can only be as accurate as the information provided by those navigation systems. Because existing navigation systems are error-prone, current robotic surgeons cannot be trusted to autonomously perform any surgical procedure. Rather, robotic surgeons are simple automated holding arms that hold an instrument. The actual drilling still needs to be performed by a surgeon.

The main problem with conventional navigation systems is that they do not determine the actual relative 3D positions and orientations between surgical tools and anatomy but rather infer those positions and orientations by tracking, with a camera, reference bodies externally affixed to tools (e.g., a drill) and anatomy. This camera, by its nature, can only see the externally fixated reference body but not the drill itself inside the bone. If either one of the reference bodies moves, or if the drill bends inside the bone, the navigation system will be oblivious to this fact and provide incorrect information, possibly resulting in harm to the patient.

It is desirable to have a navigation system that (i) does not require any additional procedures and apparatuses for navigation, and (ii) is capable of reliably determining the actual 3D positions and orientations of surgical objects (e.g., tools or implants) relative to a patient. While such a navigation system may be particularly useful for spinal surgery, it may also be used for many other surgical procedures where the spatial relation of a surgical object or implant relative to the patient must be precisely determined. It may even be used in combination with the mentioned conventional navigation system so as to provide redundant navigation information and, thus, to make the navigation highly robust and this level of safety may enable autonomous robotic surgery.

### SUMMARY OF THE INVENTION

At least the one or the other of the mentioned problems are mitigated or solved by the subject matter according to each of the independent claims. Further embodiments are described in the respective dependent claims.

Systems and methods are proposed, which require neither reference bodies nor trackers, to determine the 3D spatial relation (i.e., relative 3D position and 3D orientation) of a surgical object relative to a part of a patient's anatomy. The proposed systems and methods (implemented as computer program product) are configured to determine the 3D spatial relation based on a single X-ray image depicting at least part of the surgical object and at least a region of interest within the patient's anatomy.

Generally, a computer program product is provided including instructions which, when executed on a processing unit of a system, cause the system to receive one X-ray image, a 3D data set describing at least the region of interest, and a 3D model of the surgical object. The X-ray image is typically a 2D projection image depicting at least part of a surgical object including an anchor point as well as the region of interest within a patient's anatomy. The computer program product is configured to determine a 2D position of the anchor point in the X-ray image and an imaging direction onto the region of interest based on the X-ray image. Based on this information, a 3D position of the anchor point in a 3D coordinate system defined by the 3D data set is determined. Further, a 3D position and 3D orientation of the surgical object relative to the region of interest is determined by means of the computer program product based on the 3D model of the surgical object and the 3D position of the anchor point in the 3D coordinate system.

The surgical object may be a surgical instrument or tool like, e.g., a drill, a needle, or a chisel, or it may be an implant like, e.g., a pedicle screw, a nail, or a plate. The surgical object has a known geometric shape, which is described by the 3D model. The surgical object also has a point, henceforth and throughout this disclosure called the anchor point, whose position in the 3D model of the surgical object is known, and which can be identified in the X-ray image. The anchor point may for instance be the tip of a drill. In case of a chisel, the anchor point may be one edge or the midpoint of the chisel's blade.

The 3D data set describing part of the patient's anatomy may be generated by a computerized tomography (CT) scan or some other type of 3D X-ray scan, which may be obtained pre- or intra-operatively. The 3D data set includes the region of interest, which must be sufficiently rigid. The region of interest may be, e.g., a volume containing a bone fragment, a particular vertebra, or two neighboring vertebrae provided that these may be assumed to be sufficiently rigid. The region of interest may also be the entire 3D data set. Because the 3D data set accurately describes the anatomy of the patient at least within the region of interest, a one-to-one correspondence between points in the physical world and points in the 3D data set may be established at least within the region of interest. The 3D data set may thus define a 3D coordinate system in physical 3D space. It may be an aim to determine the surgical object's 3D position and orientation in this 3D coordinate system or, in other words, to determine the surgical object's 3D spatial relation (in the physical world) relative to the region of interest within the patient's anatomy.

According to an embodiment, in pre- or intra-operative planning, a target point in the 3D data set and within the region of interest is determined. The target point may be determined pre- or intraoperatively, either manually by the surgeon, automatically by the system, or semiautomatically by the surgeon with some automated support by the system. Hence, the target point's position in the 3D coordinate system defined by the 3D data set is known. The target point may be chosen such that the anchor point of the surgical object may be placed on (or close to) the physical location of the target point at the patient, which may be achieved by choosing the target point on a bone surface.

In another embodiment, the target point may be the start point of a target path, whose end point will be called the target end point. The target path lies within the region of interest, and its position and orientation within the 3D data set may be determined in pre- or intra-operative planning, either manually by the surgeon, automatically by the system, or semiautomatically by the surgeon with some automated support by the system. Therefore, if a target path is available, its position and orientation in the 3D coordinate system is known. For instance, when treating a vertebra, the target path may correspond to the planned drilling trajectory (typically a straight line), and the target point is located on the surface of the vertebra.

The methods disclosed herein teach how to determine the 3D position and the 3D orientation of the surgical object in the 3D coordinate system defined by the 3D data set that describes the patient's anatomy. By doing that, the relative 3D position and 3D orientation of the surgical object relative to the region of interest of the patient's anatomy is determined. Therefore, if the region of interest includes a planned target path, the relative 3D position and 3D orientation of the surgical object relative to the target path in the patient's anatomy may also be determined. It may also be an object to provide instructions in order to align the surgical object with the target path (if available), e.g., in order to drill along the target path.

Neither the target point nor the target path is necessarily required. According to an embodiment, it also possible to work with an anchor point (e.g., the tip of a drill) only. In this case, the surgeon may decide intra-operatively whether the 3D position and orientation of the surgical object relative to the patient's anatomy is satisfactory, and, if not, how it should be corrected.

It is emphasized that the 3D data set need not be segmented. In particular, it is not necessary to identify bone surfaces in the 3D data set (other than choosing the target point on a bone surface). Furthermore, the precise location and shape of the region of interest within the 3D data set need not be known and need not be explicitly determined. For instance, when treating a vertebra, a CT scan may contain this vertebra and also some neighboring vertebrae (and also surrounding soft tissue). Initially, the vertebra need not be identified in the 3D data set. A rough approximation of the region of interest within the 3D data set may be derived from the target point. The anchor point defines a 2D region of interest within the X-ray image. For subsequent computations, data points within the 2D region of interest may be weighted, where points further away from the anchor point receive a lower weighting.

The imaging direction onto the 3D region of interest depicted in the X-ray image may be determined by computing, from the 3D data set, digitally reconstructed radiographs (DRRs) for a multitude of imaging directions. Alternatively, a model of the imaged structure may be utilized for determining the imaging direction. The imaging direction may also be determined based on position sensors and/or movement sensor provided in the imaging device.

Throughout this disclosure, the term "imaging direction" (also called "viewing direction") onto an object (or region of interest) means the 3D angle at which an X-ray beam passes through a chosen point of the object (or region of interest). In other words, the imaging direction onto the region of interest describes the orientation of the X-ray machine relative to the anatomy within the region of interest.

When taking a DRR best matching the X-ray image to determine the imaging direction, it may also be possible to use a numerical optimizer to find an optimal DRR. It is not necessary to restrict the DRR to the region of interest. When assessing the best match between DRR and X-ray, the region of interest may be emphasized (e.g., with an appropriate weighting). If the X-ray image acquired suffers from distortion (i.e., it was acquired with an image intensifier rather than with a flat-panel receiver), it may be necessary to use a smaller region in the X-ray image for the DRR match (or to emphasize points close to the anchor point) because the larger the distance between points, the stronger the distortion may be. Because distortion typically affects areas further away from the central X-ray beam more strongly, it may be helpful to ensure that the anchor point lies close to the center of the X-ray image.

As discussed in detail in US2021/0248779 A1, the 3D pose (i.e., 3D position and orientation) of a thin surgical object such as a drill cannot always be uniquely determined based on a single X-ray image. Therefore, in general there may be an ambiguity in the relative 3D spatial relation between the surgical object and the patient's anatomy if only one X-ray image is available. The present disclosure teaches to resolve such an ambiguity by first determining the 3D position of the anchor point in the 3D coordinate system defined by the 3D data set. Different methods are disclosed how this can be achieved.

A method according to an embodiment, which does not use a target point, requires the anchor point to be located on a bone surface. Based on a single X-ray image, the 2D position of the anchor point is determined in the X-ray image, and the imaging direction of the X-ray image onto the region of interest may be determined from a DRR match. In 3D space, the points whose virtual projections into the X-ray image lie on the anchor point determine a line. Because the anchor point lies on the bone surface, the position of the anchor point in 3D can be found by determining the point on the line that lies on the bone surface. It is sufficient to determine the location of the bone surface along the line. A segmentation of the entire bone's surface is not necessary.

Another method establishes a correspondence between the anchor point (on the surgical object) and the target point (in the 3D data set). This is done by virtually projecting the target point into the X-ray image, which utilizes the known imaging direction onto the region of interest determined through, e.g., a DRR match.

If the location of the anchor point matches the location of the target point in the X-ray image, it may be assumed that the 3D position of the anchor point matches the 3D position of the target point in physical space. This is because the target point is chosen such that the anchor point of the surgical tool may be placed (in physical space) on the target point. Of the three coordinates of the 3D position, two degrees of freedom are determined by the X-ray image, and the third degree of freedom is determined by the a priori information that the target point and the anchor point lie on the bone surface or at a defined distance from it.

Typically, the 3D data set (e.g., the CT scan) contains scaling information (i.e., information about sizes) for the anatomy described in the data set. Moreover, a scale of the scene depicted in the X-ray may also be provided by the depicted surgical object whose exact geometric shape is known, or other surgical objects of known geometry that may be depicted in the X-ray image (e.g., an already implanted pedicle screw). This redundancy with respect to scale information may be used to differentiate between different surgical objects in the X-ray image if these are of identical geometric shape but with different sizes (e.g., drills with different diameters), by comparing the scale of the 3D data and the size of the surgical object. If the X-ray image acquired suffers from distortion, the same information (i.e., the 3D data set, the 3D model of the surgical object, or another surgical object of known geometry) may also be used to correct the distortion.

If the location of the anchor point does not match the location of the (virtually projected) target point in the X-ray image, instructions may be provided by the system how to move the surgical tool such that the anchor point is closer to the target point, after which a new X-ray may be acquired. If the location of the anchor point is in the vicinity of the target point in the X-ray image and a local model of the bone surface in the vicinity of the target point is available, the 3D position of the anchor point may be obtained, for the 2D position of the anchor point detected in the X-ray image, from the local model. This suffices to determine the 3D relative spatial relation between the surgical object and the patient's anatomy. Such a local model of the bone surface may, for instance, be a median shape of the bone, a first-order approximation of the shape of the bone, or a local segmentation of the bone surface in the vicinity of the target point. The correct model may be selected based on a labeling of the 3D data set (e.g., classifying the vertebra), which may be done automatically, semiautomatically, or manually. The size of the vicinity of the target point may be adapted based on the local model, e.g., the less variation the local model has close to the target point, the larger the vicinity may be chosen. Of course, in case that a segmentation of the 3D data set (i.e., a model of the entire bone surface) is available, this can be taken into account, which may enhance accuracy.

It is emphasized again that the precise location and shape of the 3D region of interest within the 3D data set need not be known. Moreover, it is not necessary to establish a precise correspondence between the 2D region of interest in the X-ray image and the 3D region of interest in the 3D data set. There may, however, be cases where there are several possible 3D regions of interest. For instance, in spinal surgery, there may be a plurality of 3D regions of interest because a plurality of vertebrae is to be treated. In such a case, a human surgeon may identify manually which 3D region of interest is to be treated. It may also be possible to automatically select the relevant 3D region of interest, corresponding to the anchor point indicated with the surgical tool, by performing a DRR match for each possible 3D region of interest and selecting the best such match.

The region of interest is an approximation of the area (or volume) that can be assumed to be sufficiently rigid so that the 3D data set describes the patient's anatomy within the region of interest with sufficient accuracy. For example, since a spine is flexible, individual vertebrae may move relative to each other. If the patient was moved after acquiring the 3D data set, some relative movement of individual vertebrae would have to be expected. This means that the area that may be assumed to be rigid may be larger with an intra-operative 3D X-ray (without subsequent patient movement) than with a pre-operative CT scan (with subsequent patient movement), assuming no significant movement due to breathing.

The methods taught in this disclosure may also complement existing navigation technologies. Another aspect may be a continual incorporation of information from intraoperative X-rays. While the systems and methods disclosed herein do not require a camera or other sensor for navigation, it may nevertheless (for increased accuracy and/or redundancy) be combined with a camera or other sensor (e.g., a sensor attached to the robot) for navigation. Information from the X-ray image and information from the camera or other sensor may be combined to improve the accuracy of the determination of relative spatial position and orientation, or to resolve any remaining ambiguities (which may, for instance, be due to occlusion). If the tool is held by a robot or robotic arm, information provided by the robot or robotic arm itself (e.g., about the movements it has performed) may also be considered.

Possible indications for applying this disclosure include any type of bone drilling, e.g., for insertion of a screw into a pedicle, wedge osteotomy, a screw into a sacroiliac joint, a screw connecting two vertebrae, a screw through the pelvis, a screw through the acetabulum, or a drilling for crucial ligament. The disclosed teaching may be used, e.g., for drilling, reaming, milling, chiseling, sawing, resecting, and implant positioning, hence it may also support, e.g., osteotomy, tumor resection, total hip replacement.

It may be understood that the system may comprise a processing unit and that the method may be implemented as computer program product which can be executed on that processing unit.

According to an embodiment, the system and method may cause an imaging device to generate an X-ray image or some type of 3D X-ray scan (e.g., a CT scan). Additionally or alternatively, the system and method may control the imaging device to move to a new position for generating an X-ray image from a different imaging direction. Such different imaging direction may be the appropriate imaging direction as suggested by the system. The current imaging direction may be determined on the basis of internal movement and/or position sensors of the imaging device.

It is noted that the image data of the processed X-ray image may be received directly from an imaging device, for example from a C-arm, G-arm, or biplanar 2D X-ray device, or alternatively from a database. A biplanar 2D X-ray device has two X-ray sources and receivers that are offset by any angle.

According to an embodiment, objects in the X-ray image may automatically be classified and identified in an X-ray projection image. However, an object may also be manually classified and/or identified in the X-ray projection image. Such a classification or identification may be supported by the device by automatically referring to structures that were recognized by the device.

It is noted that a processing unit may be realized by only one processor performing all the steps of the process, or by a group or a plurality of processors, which need not be located at the same place. For example, cloud computing allows a processor to be placed anywhere. For example, a processing unit may be divided into a first sub-processor that controls interactions with the user, including a monitor for visualizing results, and a second sub-processor (possibly located elsewhere) that performs all computations. The first sub-processor or another sub-processor may also control movements of, for example, a C-arm or a G-arm of an X-ray imaging device.

According to an embodiment, the device may further comprise storage means providing a database for storing, for example, X-ray images. It will be understood that such storage means may also be provided in a network to which the system may be connected, and that data related to a neural net may be received over that network. Furthermore, the device may comprise an imaging unit for generating at least one 2D X-ray image, wherein the imaging unit may be capable of generating images from different directions.

According to an embodiment, the system may comprise a device for providing information to a user, wherein the information includes at least one piece of information out of the group consisting of X-ray images and instructions regarding step of a procedure. It will be understood that such a device may be a monitor or an augmented reality device for visualization of the information, or it may be a loudspeaker for providing the information acoustically. The device may further comprise input means for manually determining or selecting a position or part of an anatomical structure in the X-ray image, such as a bone outline, for example for measuring a distance in the image. Such input means may be for example a computer keyboard, a computer mouse, or a touch screen, to control a pointing device like a cursor on a monitor screen, which may be included in the device. The device may also comprise a camera or a scanner to read the labeling of a packaging or otherwise identify a surgical object. A camera may also enable the user to communicate with the device visually by gestures or mimics, e.g., by virtually touching devices displayed by virtual reality. The device may also comprise a microphone and/or loudspeaker and communicate with the user acoustically.

It is noted that all references to C-arm movements in this disclosure always refer to a relative repositioning between C-arm and patient. Hence, any C-arm translation or rotation may in general be replaced by a corresponding translation or rotation of the patient/OR table, or a combination of C-arm translation/rotation and patient/table translation/rotation. This may be particularly relevant when dealing with extremities since in practice moving the patient's extremities may be easier than moving the C-arm. It is noted that the required patient movements are generally different from the C-arm movements, in particular, typically no translation of the patient is necessary if the target structure is already at the desired position in the X-ray image. The system may compute C-arm adjustments and/or patient adjustments. It is furthermore noted that all references to a C-arm may analogously apply to a G-arm, O-arm, or similar.

The methods and techniques disclosed herein may be used in a system that supports a human user or surgeon, or they may also be used in a system where some or all of the steps are performed by a robot. Hence, references to a "user" or "surgeon" in this patent disclosure may refer to a human user as well as a robotic surgeon, a mechanical support device, or a similar apparatus. Similarly, whenever it is mentioned that instructions are given how to adjust the C-arm, it is understood that such adjustments may also be performed without human intervention, i.e., automatically, by a robotic C-arm, by a robotic table, or they may be performed by OR staff with some automatic support. It is noted that because a robotic surgeon and/or a robotic C-arm may operate with higher accuracy than humans, iterative procedures may require fewer iterations, and more complicated instructions (e.g., combining multiple iteration steps) may be executed. A key difference between a robotic and a human surgeon is the fact that a robot may keep a tool perfectly still in between acquisition of two X-ray images.

A computer program product may preferably be loaded into the random-access memory of a data processor. The data processor or processing unit of a system according to an embodiment may thus be equipped to carry out at least a part of the described process. Further, the disclosure may relate to a computer-readable medium such as a CD-ROM on which the disclosed computer program product may be stored. However, the computer program product may also be presented over a network like the World Wide Web and can be downloaded into the random-access memory of the data processor from such a network. Furthermore, the computer program product may also be executed on a cloud-based processor, with results presented over the network.

It is noted that prior information about a surgical object (e.g., the size and type of a drill) may be obtained by simply scanning of a packaging (e.g., the barcode) or any writing on the surgical object itself, before or during surgery.

As should be clear from the above description, a main aspect is a processing of X-ray image data, allowing an automatic interpretation of visible objects. The methods described herein are to be understood as methods assisting in a surgical treatment of a patient. Consequently, the method may not include any step of treatment of an animal or human body by surgery, in accordance with an embodiment.

It will be understood that steps of methods described herein, and in particular of methods described in connection with workflows according to embodiments some of which are visualized in the figures, are major steps, wherein these major steps might be differentiated or divided into several sub-steps. Furthermore, additional sub-steps might be between these major steps. It will also be understood that only part of the whole method may constitute the invention, i.e. steps may be omitted or summarized.

It has to be noted that embodiments are described with reference to different subject-matters. In particular, some embodiments are described with reference to method-type claims (computer program product) whereas other embodiments are described with reference to apparatus-type claims (system/device). However, a person skilled in the art will gather from the above and the following description that, unless otherwise specified, any combination of features belonging to one type of subject-matter as well as any combination between features relating to different subject-matters is considered to be disclosed with this application.

The aspects defined above and further aspects, features and advantages of the present invention can also be derived from the examples of the embodiments to be described hereinafter and are explained with reference to examples of embodiments also shown in the figures, but to which the invention is not limited.

### BRIEF DESCRIPTION OF THE DRAWINGS

Fig. 1 shows a 2D X-ray projection image depicting a spine from anterior-posterior direction.
Fig. 2 shows a slice of a 3D scan of the same spine as Fig. 1, depicting a sagittal cross-section.
Fig. 3 shows a slice of the same 3D scan as Fig. 2, depicting an axial cross-section.
Fig. 4 shows an example extended workflow for performing a pedicle screw drilling.
Fig. 5 shows an example short workflow for performing a pedicle screw drilling without changing imaging direction
Fig. 6 shows an example workflow for performing a pedicle screw drilling without planning of target points.

Throughout the drawings, the same reference numerals and characters, unless otherwise stated, are used to denote like features, elements, components, or portions of the illustrated embodiments. Moreover, while the present disclosure will now be described in detail with reference to the figures, it is done so in connection with the illustrative embodiments and is not limited by the particular embodiments illustrated in the figures.

### DETAILED DESCRIPTION OF THE EMBODIMENTS

The present disclosure teaches systems and methods how to determine the 3D position and orientation of a surgical object relative to a part of a patient's anatomy, called the region of interest, which is described by a 3D data set such as a CT scan or some other type of 3D X-ray scan, based on a single X-ray image. Without additional information, the 3D pose (i.e., 3D position and orientation) of a thin surgical object such as a drill cannot always be uniquely determined based on a single X-ray image.

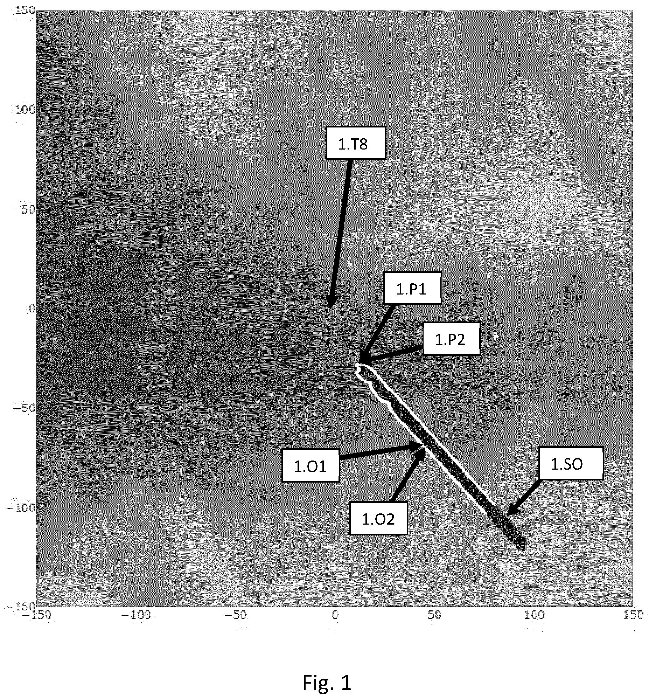

Fig. 1 shows a 2D X-ray projection image depicting a spine from anterior-posterior direction. A surgical object (1.SO) is placed such that its tip lies on the bone surface of vertebra T8 (1.T8). Two outlines of the surgical object are shown for two different 3D positions (1.P1 and 1.P2, respectively) and 3D orientations of the surgical object for which the outlines (the white solid lines marked as 1.O1 and 1.02, respectively) are more or less identical. The tip of the surgical object in 3D space lies on the line defined by the drill tip and the focal point of the X-ray machine, i.e., the epipolar line of the tip of the surgical object. Depending on the 3D position of the tip of the surgical object, the 3D orientation may be adjusted such that the projected outline of the surgical object fits its appearance in the 2D X-ray image. It is emphasized that there is an infinite number of combinations of 3D positions and corresponding 3D orientations leading to a nearly identical outline of the surgical object, of which two are depicted in Fig. 1.

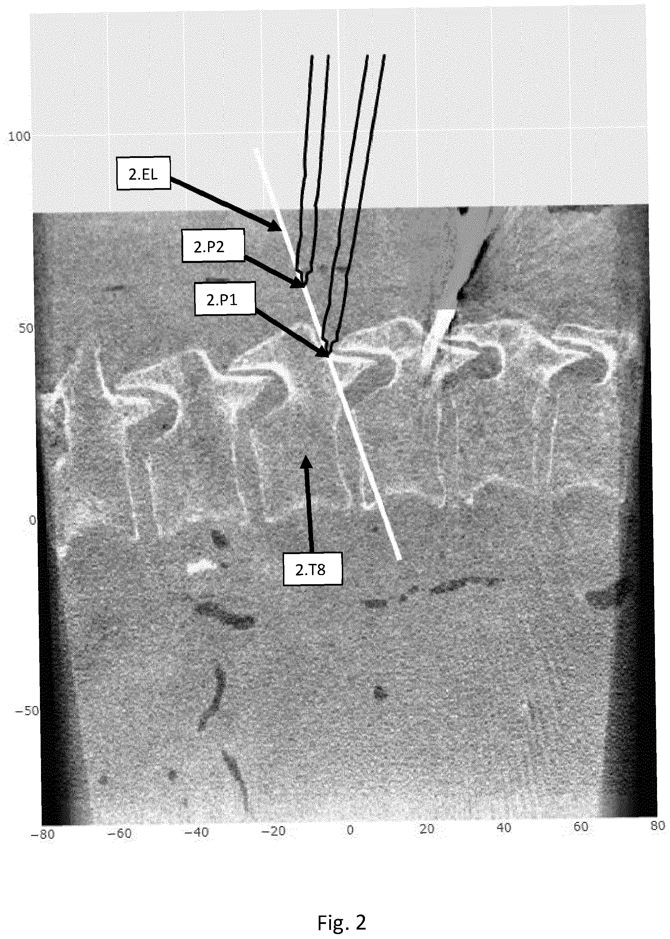

Fig. 2 shows a slice of a 3D scan of the same spine as Fig. 1, depicting a sagittal cross-section. A white line (2.EL) shows all possible 3D positions of the tip of the surgical object, corresponding to the epipolar line of the tip of the surgical object from Fig. 1. Two points on the line are chosen, one (2.P1) such that the tip of the surgical object lies on the bone surface of vertebra T8 (2.T8), and the other (2.P2) at a certain distance from the bone surface. Depending on the 3D position of the surgical object it is clearly visible that the 3D orientations of the surgical object differ, but both leading to nearly identical outlines in the X-ray projection image in Fig. 1.

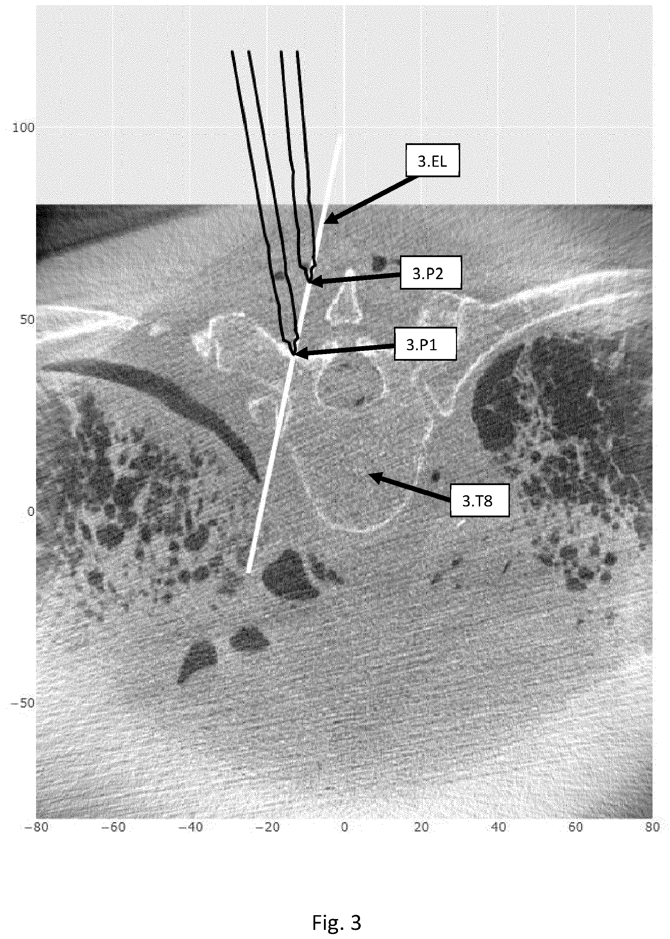

Fig. 3 shows a slice of the same 3D scan as Fig. 2, depicting an axial cross-section. A white line (3.EL) shows all possible 3D positions of the tip of the surgical object, corresponding to the line 2.EL from Fig. 2. As in Fig. 2, two points on the line are chosen, one (3.PI) such that the tip of the surgical object lies on the bone surface of vertebra T8 (3.T8), and the other (3.P2) at a certain distance from the bone surface. Again, it is visible that the 3D orientations of the surgical object differ, but both leading to nearly identical outlines in the X-ray projection image in Fig. 1.

In other words, Figures 1 to 3 show two different 3D constellations with two different drill poses, which lead to an identical (or nearly identical) X-ray projection image. The two drill constellations differ in the imaging depth (which is the distance from the image plane, i.e., from the X-ray receiver) and tilt (i.e., 3D orientation). However, if it is possible to remove the ambiguity regarding imaging depth (due to some a priori information), the drill pose relative to the patient's anatomy may be uniquely determined.

The present disclosure teaches to remove the ambiguity regarding imaging depth by establishing a correspondence between the anchor point of the surgical object and the target point (whose position in the 3D coordinate system defined by the 3D data set is known), utilizing the a priori information that the target point and the anchor point lie on the bone surface or at a defined distance from it. This may be done based on a single X-ray image.

Alternatively, the ambiguity regarding imaging depth may also be removed without using any target point. According to an embodiment, the tip of a drill (i.e., the anchor point) is placed on a bone surface near the intended drilling path (i.e., within the region of interest), and an X-ray image is acquired. The drill tip's position is detected in the X-ray image. For the imaging direction onto the region of interest as determined based on a DRR match of the 3D data set to the X-ray image, the possible drill tip positions in 3D space whose virtual projections into the X-ray image lie on the detected anchor point determine a line (a so-called epipolar line) in the 3D coordinate system defined by the 3D data set. Because of the a priori knowledge that the drill tip lies on the bone surface, the position of the drill tip in the coordinate system defined by the 3D data set can be found by determining the point on the line that intersects (i.e, lies on) the bone surface. Finding the bone surface along the line may be done automatically, e.g., by evaluating the Hounsfield values in the 3D data set along the line (or in a vicinity around the line) and searching for strong gradients. Of course, if a local segmentation of the bone surface in the relevant region is already available, this may be used for intersecting the line.

It may be possible to increase the accuracy of determining the spatial relation between the surgical object and the region of interest by acquiring further X-ray images from the current and/or different imaging directions.

It is also possible to determine the 3D spatial relation of the surgical object relative to the region of interest after the surgical object has been inserted into the patient, i.e., the anchor point is no longer located on the bone surface. This is of interest, for instance, when performing a drilling. In such a case, at a first point in time, an X-ray image is acquired, from which the drilling start point in the coordinate system of the 3D data is determined, which is assumed to be the 3D position of the drill tip (i.e., the anchor point) at the first point in time. After advancing the tool into the patient, at a second point in time, a further X-ray image is acquired. The ambiguity regarding the 3D drill pose may now be resolved based on the assumption that the drill axis, as determined at the second point in time, runs through the drilling start point as determined at the first point in time. This assumption requires that the drilling actually started at the anchor point as determined at the first point in time. This assumption may be particularly easy to justify if a surgical robot is used because this eliminates unintentional movements of the surgical object, and movements of the patient may be detected by the single-image registration.

This procedure may be repeated, possibly also acquiring X-ray images from other imaging directions for increased accuracy.

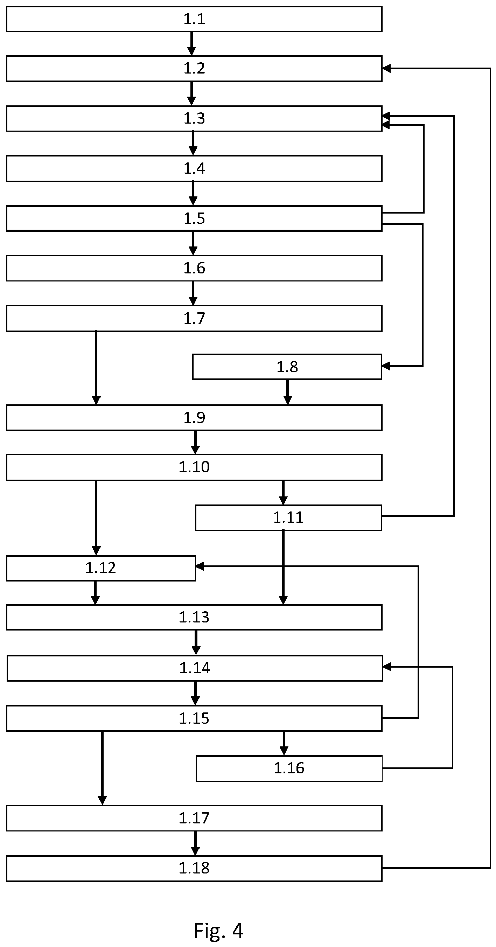

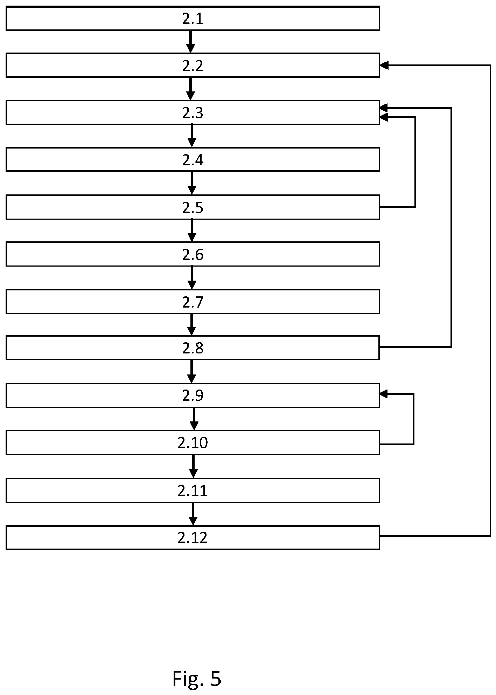

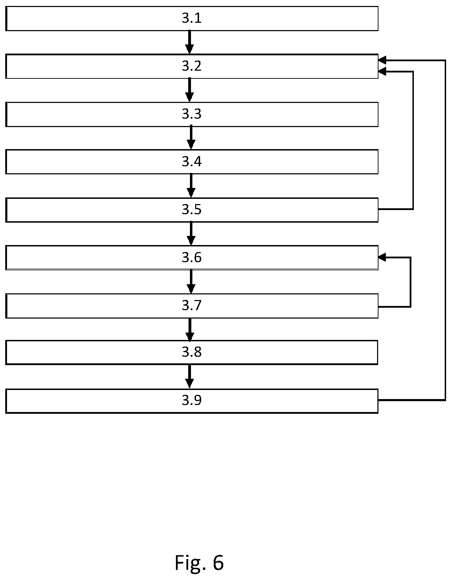

According to an embodiment, such a system may be particularly useful for spinal surgery. The following three workflows are examples how this system may be used to perform a pedicle screw drilling for spinal fusion. The first workflow (cf. Fig. 4) is an extended workflow for increased accuracy, requiring a plurality of X-ray images from different imaging directions. The second workflow (cf. Fig. 5) is a shorter workflow without changing imaging direction and the third workflow (cf. Fig. 6) is a workflow without planning of target points.

### Example workflow (extended workflow) for performing a pedicle screw drilling (cf. Fig. 4)

It is noted that the numbering of the steps in the workflow of Fig. 4 is a combination of a "1." indicating that the workflow is the first one out of three, followed by an actual number of the step as used in the following description of the workflow.
1. The surgeon and/or the system performs a pre-operative planning in the 3D data set (e.g., a CT scan or 3D X-ray scan) by determining a target path, i.e., the planned drilling trajectory, within the 3D data set, where the target point is the starting point of the intended drilling trajectory, which is located on the bone surface of the pedicle, and the target end point is the end point of the intended drilling trajectory.
2. The surgeon positions the drill tip on the dorsal bone surface in approximate vicinity of the pedicle to be drilled in (in case the relative position of drill and bone is already known, the drill tip may be positioned more precisely).
3. The surgeon acquires an X-ray image (e. g., approximately in anterior-posterior (AP) imaging direction), preferably holding the drilling machine in such a way that the surgeon's hand is not in the X-ray beam. If an imaging direction were to lead to the surgeon's hand being in the X-ray beam, the system may provide instructions for a different imaging direction.
4. The system detects the position of the drill tip (i.e., the anchor point) in the X-ray image. The system determines the imaging direction onto the region of interest in the X-ray image by computing, from the 3D data set, digitally reconstructed radiographs (DRRs) for a multitude of imaging directions. The DRR best matching the X-ray image is taken to determine the imaging direction. It is not necessary to restrict the DRR to the region of interest. The system may use a weighting function to match the important regions within the 2D region of interest more accurately (e. g., in case of bad image quality), where the 2D region of interest is determined by the drill tip position in the X-ray. The weighting function may also depend on the C-arm type (flat-panel vs. image intensifier), the type of vertebra (cervical/thoracic/lumbal) and/or the type of 3D data set (pre-operative vs. intra-operative). The system determines a virtual projection of the target point into the X-ray image based on the imaging direction, which projection may be displayed as an overlay in the X-ray image.
5. If the 2D distance between the drill tip (i.e., the anchor point) and the virtual projection of the target point in the X-ray image is below a threshold (e. g., 2 mm; the threshold may depend on the type of vertebra), the system continues with Step 8. Otherwise, the system gives an instruction to reposition the drill tip to move it close to the virtual projection of the target point. The surgeon decides whether he/she
   a. follows the instruction and returns to Step 3, or
   b. proceeds with the current drill tip position and continues with Step 6.
6. The surgeon acquires an X-ray image from another imaging direction (e. g., lateral or a direction between AP and lateral, or between AP and axial) while ensuring that the drill tip position does not move compared to the previous X-ray image.
7. The system determines the new imaging direction by performing a new DRR matching between the 3D data set and the current X-ray image as described above. This registers the current X-ray image with the previous X-ray image. The system detects the drill tip in the current X-ray image and determines its 3D position in the coordinate system of the 3D data set based on epipolar geometry and utilizing the known 3D model of the drill. If it can be ensured that the drill was not moved or tilted in between acquiring the X-ray images in Steps 3 and 6 (e.g., if a robot holds the drill), the matched drill can be used to enhance the accuracy of image registration; in this case, continue with Step 9.
8. The system determines the 3D drill tip position in the coordinate system of the 3D data set based on the detected drill tip in the X-ray image by applying the knowledge that both the drill tip and the target point lie on the bone surface. In case the drill tip has a remaining 2D distance to the target point, the determination of the 3D drill tip position may take into account a local model of the vertebra's surface in the vicinity of the target point. The local model may be derived from a model of the vertebra in case the vertebra to be drilled has been already classified (i.e., which vertebral level, e.g., L3, L4, etc.).
9. Based on the determined 3D drill tip position in the coordinate system of the 3D data set, the system determines the 3D position and orientation of the drill in the coordinate system of the 3D data set.
10. If the deviation between the 3D drill orientation and the target path is below a threshold (e. g., less than 2 °), continue with Step 12. Otherwise, the system gives an instruction to adjust the drill angle.
11. The surgeon adjusts the drill angle and may return to Step 3 (without the need of changing the imaging direction) or directly continues/commences drilling continues with Step 13.
12. The system calculates the (remaining) drilling depth and gives a corresponding drilling instruction.
13. The surgeon follows the instruction (e. g., by drilling a given distance). A new X-ray image may be acquired (without the need of changing the imaging direction) whenever a confirmation of the drilling trajectory is desired or advised by the system (e.g., after drilling a certain distance or if drilling close to critical anatomy).
14. The system performs matchings, detections, etc. as described above. Based on the actual drilling start point (this may be, e g., the position of the drill tip as shown in the X-ray image when the first drilling instruction was given), the system determines the 3D position and 3D orientation of the drill.
15. If the remaining drilling distance is below a threshold (e. g., 1 mm), continue with Step 17. If the deviation between the 3D drill orientation and the target path is below a threshold (e. g., 1 °), return to Step 12. Otherwise, the system gives an instruction to adjust the drill angle. If there was a too deep drilling, system may provide according warning
16. The surgeon follows the instruction, acquires a new X-ray image, and returns to Step 14.
17. The drilling procedure for the current pedicle is completed.
18. For performing the next pedicle drilling return to Step 2.

It is noted again that all references to a "surgeon" in this workflow may refer to a human user as well as a robotic surgeon, a mechanical support device, or a similar apparatus.

In general, particularly in addition to each of the example workflows as disclosed herein, the system may also provide support for performing skin incisions. This may be particularly useful if a surgical robot or a robotic arm is employed. According to an embodiment, a surgical tool (e.g., a scalpel) is aligned with a target trajectory, but with a sufficiently large distance from the target point such that the surgical tool does not yet touch the skin. Using a soft-tissue protection sleeve, the robot or robotic arm may then move the surgical tool along the target trajectory toward the target point until it encounters some resistance (i.e., it touches the skin). Resistance may be detected automatically, e.g., by a pressure sensor. An incision may be made through the soft-tissue protection sleeve. After making the incision, the surgical tool may be exchanged (e.g., switching to a drill while keeping the soft-tissue protection sleeve in place) and the surgical tool may be advanced until it encounters a larger resistance (i.e., it touches the bone surface). Resistance may be detected automatically, e.g., by a pressure sensor. After completing the drilling for one pedicle, the procedure may be repeated for the next pedicle.

It may be helpful if the robot or robotic arm can automatically switch surgical tools (possibly within a soft-tissue protection sleeve), advance or withdraw the soft-tissue protection sleeve, and drill through the soft-tissue protection sleeve (possibly using an oscillating drilling mode). It may be advantageous to specifically design a soft-tissue protection sleeve or adapter suitable for a robot. The design of such a sleeve may also take into account detectability in an X-ray image. For instance, it may be advantageous to make the sleeve from radiotranslucent material or partially from radiotranslucent material (e.g., in the region covering the tip of a drill) such that the surgical object (e.g., the drill and especially its tip) is visible in an X-ray image even when covered by the sleeve. It may also be advantageous to incorporate specific features into the surgical object and/or the sleeve that may be used to make the detection of the surgical object in an X-ray image easier.

According to an embodiment, using a surgical robot may also allow to address the movement between individual vertebrae that are due to the patient breathing. This breathing may be modeled and detected by a pressure sensor. The robot may be capable of keeping the pressure on the vertebra constant when it is not drilling.

This procedure may also be applied for determining the position of a chisel, e.g., during a wedge osteotomy, where the midpoint of a chisel is used as the anchor point.

A similar procedure may also be applicable when using a soft-tissue protection sleeve not made from radiotranslucent material, where the anchor point (e.g., the tip of the drill) may not be visible in the X-ray image. In such a case, instead of using the (not visible) anchor point, a virtual anchor point may be used. This virtual anchor point may be determined based on a previously acquired X-ray image where the anchor point was visible and based on the assumption that there has been no movement of the anchor point between the generation of the current X-ray image and the generation of the previously acquired X-ray image. Based on the current X-ray image, a plane is defined by the axis of the sleeve and the center of the X-ray beam. The virtual anchor point may be determined by orthogonally projecting the anchor point of the previous X-ray image onto that plane. The virtual anchor point may also be determined by choosing the closest point from the anchor point of the previous X-ray image to a contour that lies within the plane (e.g. intersection of the plane with a model of a bone or a partial 3D segmentation of the bone surface) to ensure that the virtual anchor point is positioned on the bone surface.

Another application of the procedure may be a tumor resection. In this case, a preoperatively acquired MRI scan (e.g., including an outline of a tumor or a planned resection volume) may be fused with a pre- or intraoperatively acquired 3D X-ray scan, where one or more than one target point is identified in the MRI scan and/or the 3D X-ray scan. The surgical instrument may be a resection instrument having an anchor point. By determining the relative 3D position and 3D orientation of the resection instrument and the tumor outline (or planned resection volume), precise 3D navigation instructions may be provided also for the case when several target points have been defined in the planning. Resection may also take place at a certain distance from a target point. Using a robot may also lead to enhanced accuracy. A robot may receive relative 3D positioning information from e.g. internal sensory devices, so the 3D position and 3D orientation of the instrument relative to the resection volume is available at any point in time after the last determination based on acquisition of an X-ray image. A validation of this information is possible at any point in time by acquisition of an additional x-ray image even without using anchor point or target point: Based on the available information from the positioning sensory devices and the last determination of 3D position and 3D orientation of the instrument relative to the resection volume based on the last X-ray image, a virtual X-ray image is generated assuming the imaging direction has not changed. After acquisition of the additional x-ray image from the same imaging direction, the additional X-ray image is compared to the virtual x-ray image and an algorithm decides whether the similarity is high enough (positioning information still valid) or not (there may have been an unknown movement. In latter case a new image with determination of anchor point and target point may be needed.

This procedure may also be combined with a real-time navigation and tracking system. As the procedure is redundant to existing technology and in addition provides a high level of diversification such a combination would make the navigation highly robust and this level of safety may enable autonomous robotic surgery.

### Example workflow ( no changes of imaging direction ) for performing a pedicle screw drilling (cf. Fig. 5)

It is noted that the numbering of the steps in the workflow of Fig. 5 is a combination of a "2." indicating that the workflow is the second one out of three examples, followed by an actual number of the step as used in the following description of the workflow.
1. The surgeon and/or the system performs a pre-operative planning in the 3D data set (e.g., a CT scan or 3D X-ray scan) by determining a target point, within the 3D data set, where the target point is the starting point of the intended drilling trajectory, which is located on the bone surface of the pedicle.
2. The surgeon positions the drill tip on the dorsal bone surface in approximate vicinity of the pedicle to be drilled in (in case the relative position of drill and bone is already known, the drill tip may be positioned more precisely).
3. The surgeon acquires an X-ray image (e. g., approximately in roughly anterior-posterior (AP) imaging direction), preferably holding the drilling machine in such a way that the surgeon's hand is not in the X-ray beam. If an imaging direction were to lead to the surgeon's hand being in the X-ray beam, the system may provide instructions for a different imaging direction.
4. The system detects the position of the drill tip (i.e., the anchor point) in the X-ray image. The system determines the imaging direction onto the region of interest in the X-ray image by computing, from the 3D data set, digitally reconstructed radiographs (DRRs) for a multitude of imaging directions (This may have been performed already before acquisition of the X-ray image). The DRR best matching the X-ray image is taken to determine the imaging direction. It is not necessary to restrict the DRR to the region of interest. The system may use a weighting function to match the important regions within the 2D region of interest more accurately (e. g., in case of bad image quality), where the 2D region of interest is determined by the drill tip position in the X-ray. The weighting function may also depend on the C-arm type (flat-panel vs. image intensifier), the type of vertebra (cervical/thoracic/lumbal) and/or the type of 3D data set (pre-operative vs. intra-operative). The system determines a virtual projection of the target point into the X-ray image based on the imaging direction, which projection may be displayed as an overlay in the X-ray image.
5. If the 2D distance between the drill tip (i.e., the anchor point) and the virtual projection of the target point in the X-ray image is below a threshold (e. g., 1 mm; the threshold may depend on the type of vertebra), the system continues with Step 6. Otherwise, the system gives an instruction to reposition the drill tip to move it close to the virtual projection of the target point. The surgeon follows the instruction and returns to Step 3.
6. The system determines the 3D drill tip position in the coordinate system of the 3D data set based on the detected drill tip in the X-ray image by applying the knowledge that both the drill tip and the target point lie on the bone surface. In case the drill tip has a remaining 2D distance to the target point, the determination of the 3D drill tip position may take into account a local model of the vertebra's surface in the vicinity of the target point. The local model may be derived from a model of the vertebra in case the vertebra to be drilled has been already classified (i.e., which vertebral level, e.g., L3, L4, etc.).
7. Based on the determined 3D drill tip position in the coordinate system of the 3D data set, the system determines the 3D position and orientation of the drill in the coordinate system of the 3D data set. The system displays the current position in different views based on the 3D data set, e.g. axial view and sagittal view, it may prolong the trajectory of the drill in order to better visualize the corresponding drilling path.
8. The surgeon adjusts the drill angle and may return to Step 3 (without the need of changing the imaging direction) or directly commences/continues drilling continue with Step 9.
9. A new X-ray image may be acquired (without the need of changing the imaging direction) whenever a confirmation of the drilling trajectory is desired or advised by the system (e.g., after drilling a certain distance or if drilling close to critical anatomy).
10. The system performs matchings, detections, etc. as described above. Based on the actual drilling start point (this may be, e g., the position of the drill tip as shown in the X-ray image when the first drilling instruction was given), the system determines the 3D position and 3D orientation of the drill. The system displays the current position of the drill in different views based on the 3D data set, e.g. axial view and sagittal view. If the surgeon does not accept the current position as being ok, he/she corrects may correct the angulation (e.g. while drill bit is running) and proceeds to step 9.
11. The surgeon decides if drilling procedure for the current pedicle is completed.
12. For performing the next pedicle drilling return to Step 2.

### Example workflow (no planning of target points) for performing a pedicle screw drilling (cf. Fig. 6)

Like in the previous workflows, the numbering of the steps in the workflow of Fig. 6 is a combination of a "3." indicating that the example workflow is the third one out of three, followed by an actual number of the step as used in the following description of the workflow.
1. The surgeon positions the drill tip on the dorsal bone surface in approximate vicinity of the pedicle to be drilled in
2. The surgeon acquires an X-ray image (e. g., approximately in roughly anterior-posterior (AP) imaging direction), preferably holding the drilling machine in such a way that the surgeon's hand is not in the X-ray beam. If an imaging direction were to lead to the surgeon's hand being in the X-ray beam, the system may provide instructions for a different imaging direction.
3. The system detects the position of the drill tip (i.e., the anchor point) in the X-ray image. The system determines the imaging direction onto the region of interest in the X-ray image by computing, from the 3D data set, digitally reconstructed radiographs (DRRs) for a multitude of imaging directions (This may have been performed already before acquisition of the X-ray image). The DRR best matching the X-ray image is taken to determine the imaging direction. It is not necessary to restrict the DRR to the region of interest. The system may use a weighting function to match the important regions within the 2D region of interest more accurately (e. g., in case of bad image quality), where the 2D region of interest is determined by the drill tip position in the X-ray. The weighting function may also depend on the C-arm type (flat-panel vs. image intensifier), the type of vertebra (cervical/thoracic/lumbal) and/or the type of 3D data set (pre-operative vs. intra-operative). The system determines a line in the 3D data set defined by the anchor point and the center of the beam. The system determines a point on this line where the line intersects with the bone surface (e.g. based on starting at the most posterior point of the line, observing the voxels on this line and determining the voxel with highest gradient of greyscale on this line and possibly in the surrounding of this point). This point defines the position of the anchor point in the 3D data set.
4. Based on the determined 3D drill tip position in the coordinate system of the 3D data set, the system determines the 3D position and orientation of the drill in the coordinate system of the 3D data set. The system displays the current position in different views based on the 3D data set, e.g. axial view and sagittal view, it may prolong the trajectory of the drill in order to better visualize the corresponding drilling path.
5. The surgeon may adjust the drill tip position and/or angle and may return to Step 2 (without the need of changing the imaging direction) or directly commences/continues drilling continue with Step 6.
6. A new X-ray image may be acquired (without the need of changing the imaging direction) whenever a confirmation of the drilling trajectory is desired or (e.g. in case of using a robot for drilling) advised by the system (e.g., after drilling a certain distance or if drilling close to critical anatomy).
7. The system performs matchings, detections, etc. as described above. Based on the actual drilling start point (this may be, e g., the position of the drill tip as shown in the X-ray image when the first drilling instruction was given), the system determines the 3D position and 3D orientation of the drill. The system displays the current position of the drill in different views based on the 3D data set, e.g. axial view and sagittal view. If the surgeon does not accept the current position as being ok, he/she corrects may correct the angulation (e.g. while drill bit is running) and proceeds to step 6.
8. The surgeon decides if drilling procedure for the current pedicle is completed.
9. For performing the next pedicle drilling return to Step 2.

In the following, examples of methods are provided, wherein these methods are implemented as computer program products.
1. An example of a computer program product including instructions which, when executed on a processing unit of a system, cause the system
   to determine a 3D position and 3D orientation of object 1 relative to object 2 at a first point in time,
   to determine a 3D position and 3D orientation of object 1 relative to object 2 at a second point in time based on information received from a real-time tracking system,
   to receive a 2D X-ray image generated at the second point in time by an X-ray imaging device, the 2D X-ray image depicting at least a part of the object 1 and at least a part of the object 2,
   to receive either 3D data containing an object 1 or a 3D model of object 1,
   to receive either 3D data containing an object 2 or a 3D model of object 2,
   to generate a virtual projection image based on
      (i) the 3D data containing object 1 or the 3D model of object 1,
      (ii) the 3D data containing object 2 or the 3D model of object 2, and
      (iii) the 3D position and 3D orientation of object 1 relative to object 2 at the second point in time based on information received from a real-time tracking system, and
   to compare the 2D X-ray image and the virtual projection image.
2. The computer program product of example 1, wherein the 3D position and 3D orientation of object 1 relative to object 2 at the second point in time based on information received from a real-time tracking system is validated based on the comparison of the 2D X-ray image and the virtual projection image.
3. The computer program product of example 2, wherein the comparison is based on similarity thresholds based on different weighting of different 2D regions of the image.
4. The computer program product of any one of examples 1 to 3, wherein an imaging direction of the 2D X-ray image is determined based on at least one out of the group:
   a generation of a plurality of virtual projection images of object 1 and selecting the virtual projection image out of the plurality of images with best fit to object 1 in the 2D X-ray image,
   an assumption that the imaging direction of the X-ray imaging device is the same as determined at a previous point in time,
   tracking of the movement of the X-ray imaging device by means of a real-time tracking system.
5. The computer program product of any one of examples 1 to 4, wherein the determination of the 3D position and 3D orientation of object 1 relative to object 2 at a first point in time is based on at least one out of the group consisting of acquisition of a plurality of surface points, acquisition of a plurality of 2D X-ray images with a tracked X-ray imaging device, acquisition of intra-operative 3D scan with a tracked imaging device, acquisition of at least one 2D X-ray image including an object having an anchor point.
6. The computer program product of any one of examples 1 to 5, wherein the real-time tracking system is at least one out of the group consisting of positioning and/or movement sensory devices of a robot, a navigation system with optical trackers, a navigation system with infrared trackers, a navigation system with EM tracking, a navigation system utilizing a 2D camera, a navigation system utilizing Lidar, a navigation system utilizing a 3D camera, a navigation system including a wearable tracking element like augmented reality glasses,
7. The computer program product of example 2, wherein the 3D position and 3D orientation of object 1 relative to object 2 at the second point in time is further validated based on another determination of the 3D position and 3D orientation of object 1 relative to object 2 at the second point in time, wherein the other determination is based on a single 2D X-ray image taking into account a specific point at one of the objects.

A system comprising a processing unit configured to execute the computer program product according to any one of the preceding examples may further comprise a real-time navigation and tracking system, wherein the real-time navigation and tracking system is configured to provide 3D position and 3D orientation of a first object relative to a second object. The system may further comprise a robotic device for holding and moving one of the objects. The system may further include an X-ray imaging device.

A method for validating a 3D position and 3D orientation of an object 1 relative to an object 2, may comprise the steps of
determining a 3D position and 3D orientation of object 1 relative to object 2 at a first point in time,
determining a 3D position and 3D orientation of object 1 relative to object 2 at a second point in time based on information received from a real-time tracking system,
receiving a 2D X-ray image generated at the second point in time by an X-ray imaging device, the 2D X-ray image depicting at least a part of the object 1 and at least a part of the object 2,
receiving either 3D data containing an object 1 or a 3D model of object 1,
receiving either 3D data containing an object 2 or a 3D model of object 2,
generating a virtual projection image based on
   (i) the 3D data containing object 1 or the 3D model of object 1,
   (ii) the 3D data containing object 2 or the 3D model of object 2, and
   (iii) the 3D position and 3D orientation of object 1 relative to object 2 at the second point in time, and
comparing the 2D X-ray image and the virtual projection image.

While embodiments have been illustrated and described in detail in the drawings and aforegoing description, such illustrations and descriptions are to be considered illustrative or exemplary and not restrictive, and the invention is not limited to the disclosed embodiments.

Other variations to the disclosed embodiments can be understood and effected by those skilled in the art in practising the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims.

The mere fact that certain measures are recited in mutually different dependent claims does not indicate that a combination of these measures can not be used to advantage.

## Claims

1. A computer program product including instructions which, when executed on a processing unit of a system, cause the system
- to receive an X-ray image generated at a first point in time, wherein the X-ray image is a 2D projection image depicting at least part of a surgical object including an anchor point and a region of interest within a patient's anatomy,
- to receive a 3D data set describing the region of interest and defining a 3D coordinate system,
- to receive a 3D model of the surgical object, wherein the position of the anchor point is known in the 3D model,
- to determine a 2D position of the anchor point in the X-ray image,
- to determine an imaging direction onto the region of interest based on the X-ray image,
- to determine a 3D position of the anchor point in the 3D coordinate system at the first point in time based on
i. the 2D position of the anchor point in the X-ray image and
ii. the imaging direction onto the region of interest,
- to determine a 3D position and 3D orientation of the surgical object relative to the region of interest at the first point in time based on
i. the 3D model of the surgical object, and
ii. the 3D position of the anchor point in the 3D coordinate system at the first point in time.

2. The computer program product of claim 1, including further instructions causing the system
- to determine a line in the 3D coordinate system, the line including points whose virtual projections into the X-ray image are lying on the anchor point in the X-ray image, and
- to determine a point of the line lying on a bone surface within the region of interest, wherein the determination of the 3D position of the anchor point in the 3D coordinate system at the first point in time is further based on the point of the line lying on the bone surface.

3. The computer program product of any one of claims 1 or 2, wherein the 3D data set includes a target point whose position in the 3D coordinate system is known, wherein the computer program product includes further instructions causing the system to determine a virtual projection of the target point into the X-ray image, and wherein the determination of the 3D position of the anchor point in the 3D coordinate system at the first point in time is further based on a 2D position of the virtual projection of the target point relative to the 2D position of the anchor point in the X-ray image at the first point in time.

4. The computer program product of claim 3, wherein the 2D position of the anchor point in the X-ray image at the first point in time and the 2D position of the virtual projection of the target point in the X-ray image are treated as being identical.

5. The computer program product of any one of claims 3 or 4, wherein the determination of the 3D position and 3D orientation of the surgical object relative to the region of interest is further based on a distance of the target point from a bone surface within the region of interest and on a distance of the anchor point from the bone surface within the region of interest.

6. The computer program product of claim 5, wherein the distance of the target point from the bone surface within the region of interest is treated as being zero, and wherein the distance of the anchor point from the bone surface within the region of interest is treated as being zero.

7. The computer program product of any one of claims 1 to 6, wherein the computer program product includes further instructions causing the system
- to receive a new X-ray image generated at a second point in time, wherein the new X-ray image is a 2D projection image depicting at least part of the surgical object and the region of interest, and
- to determine an imaging direction of the new X-ray image,
and wherein the determination of the 3D position and 3D orientation of the surgical object relative to the region of interest at the second point in time is based on
i. the 3D model of the surgical object,
ii. the imaging direction of the new X-ray image,
iii. the 3D position of the anchor point in the 3D coordinate system at the first point in time, and
iv. the assumption that an axis of the surgical object at the second point in time runs through the 3D position of the anchor point in the 3D coordinate system at the first point in time.

8. The computer program product of any one of claims 1 to 6, wherein the computer program product includes further instructions causing the system
- to receive a new X-ray image generated at a second point in time, wherein the new X-ray image is a 2D projection image depicting at least part of the surgical object and at least part of the region of interest, and
- to determine an imaging direction of the new X-ray image,
- to determine an expected 3D position and 3D orientation of the surgical object relative to the region of interest at the second point in time based on
(i) the 3D position and 3D orientation of the surgical object relative to the region of interest at the first point in time, and
(ii) information about movements of the surgical object between the first point in time and the second point in time,
- to generate a virtual projection image based on the determined imaging direction of the new X-ray image and including the surgical object at the expected 3D position and 3D orientation of the surgical object relative to the region of interest at the second point in time, and
- to compare the virtual projection image and the new X-ray image so as to validate the 3D position and 3D orientation of the surgical object relative to the region of interest at the second point in time.

9. The computer program product of any one of claims 7 and 8, wherein the computer program product includes further instructions causing the system
- to receive an other X-ray image generated from an other imaging direction at a third point in time, wherein the other X-ray image is a 2D projection image depicting at least part of the surgical object and the region of interest, and wherein the other imaging direction differs from the imaging direction of the new X-ray image generated at the second point in time,
- to determine the other imaging direction of the other X-ray image, and
- to determine the 2D position of the anchor point in the other X-ray image, and wherein the determination of the 3D position and 3D orientation of the surgical object relative to the region of interest at the third point in time is based on
i. the imaging direction of the new X-ray image at the second point in time,
ii. the other imaging direction of the other X-ray image at the third point in time,
iii. the 2D position of the anchor point in the other X-ray image, and
iv. the assumption that the 3D position of the anchor point at the second point in time is the same at the third point in time.

10. The computer program product of any one of claims 1 to 9, wherein a distortion in the X-ray image is corrected based on at least one out of the group of the 3D data set, the 3D model of the surgical object, another surgical object of known geometry.

11. The computer program product of any one of claims 1 to 10, wherein the computer program product includes further instructions causing the system to provide instructions to a user as to how to adjust at least one out of the group of position of the surgical object and 3D orientation of the surgical object.

12. The computer program product of any one of claims 1 to 11, wherein the knowledge of the position of the target point is obtained automatically.

13. The computer program product of any one of claims 3 to 12, wherein determining the relative 3D position and 3D orientation of the surgical object relative to the region of interest is further based on a local model of the bone surface in the vicinity of the target point.

14. A system comprising a processing unit configured to execute the computer program product according to any one of the preceding claims.

15. The system of claim 14 further comprising
a robotic device to which the surgical object is attached, and
a real-time navigation and tracking system,
wherein the 3D position and 3D orientation of the surgical object relative to the region of interest is determined based on both the computer program product of any one of claims 1 to 13 and on information received from the real-time navigation and tracking system.
